# EUROPEAN PATENT SPECIFICATION

(11) **EP 2 773 753 B1**
(45) Date of publication and mention of the grant of the patent: **17.10.2018**
(21) Application number: 12788227.2
(22) Date of filing: 31.10.2012
(51) Int. Cl.: A23L 29/00, C12N 9/10

(54) **A LIQUID ENZYME FORMULATION AND A PROCESS FOR ITS PREPARATION**
FLÜSSIGE ENZYMFORMULIERUNG UND VERFAHREN ZU SEINER HERSTELLUNG
FORMULATION ENZYMATIQUE LIQUIDE ET SON PROCÉDÉ DE PRÉPARATION

(30) Priority: 01.11.2011 FI 20116074
(43) Date of publication of application: 10.09.2014
(73) Proprietor: Valio Ltd, 00370 Helsinki (FI)
(72) Inventor: RAJAKARI, Kirsi, 00370 Helsinki (FI); HOTAKAINEN, Kai, 00370 Helsinki (FI); MYLLÄRINEN, Päivi, 00370 Helsinki (FI)
(74) Representative: Kolster Oy Ab
(86) International application number: PCT/FI2012/051048
(87) International publication number: WO 2013/064736

(56) References cited:
- EP-A1- 1 543 732
- EP-A1- 2 251 421
- US-A- 5 340 734
- YU-JEN YU ET AL: "Overproduction of soluble recombinant transglutaminase from Streptomyces netropsis in Escherichia coli", APPLIED MICROBIOLOGY AND BIOTECHNOLOGY, SPRINGER, BERLIN, DE, vol. 81, no. 3, 20 September 2008 (2008-09-20), pages 523-532, XP019654175, ISSN: 1432-0614, DOI: 10.1007/S00253-008-1688-7
- Pierce Pierce Biotechnology, Inc.: "Protein stability and storage", www.piercenet.com , 1 September 2005 (2005-09-01), XP007921448, Retrieved from the Internet: URL:http://sites.bio.indiana.edu/~chenlab/ protocol_files/protein_storage.pdf [retrieved on 2013-01-17]
- S. L. BRADBURY ET AL: "Glycerol as an Enzyme-Stabilizing Agent: Effects on Aldehyde Dehydrogenase", PROCEEDINGS OF THE NATIONAL ACADEMY OF SCIENCES, vol. 69, no. 9, 1 September 1972 (1972-09-01), pages 2373-2376, XP055049969, ISSN: 0027-8424, DOI: 10.1073/pnas.69.9.2373

## Description

### FIELD OF THE INVENTION

The present invention relates to a liquid enzyme formulation, particularly to a liquid and stable formulation comprising a crosslinking enzyme and/or an enzyme modifying milk proteins. Particularly the present invention relates a liquid and stable transglutaminase formulation. In addition, the present invention relates to a method for preparing a liquid enzyme formulation.

### BACKGROUND OF THE INVENTION

Enzyme formulations comprising a crosslinking enzyme and/or an enzyme modifying milk proteins, such as laccase, tyrosinase, peroxidase, sulfhydryl oxidase or glucose oxidase are commercially available both in powder and liquid formulations. However, transglutaminase and protein glutaminase products are currently in the market only in powder form. The use of a powdered enzyme product is not totally accepted due to the dust formation in all production plants. The health hazards resulting from the dusting have incurred concerns among the workers, especially.

A transglutaminase derived from *Streptoverticillium mobaraense* strain and a process for its preparation has been disclosed in the European patent No. 0 379 606 B1. Further, a method for the production of a transglutaminase using a gene isolated from *Streptomyces lydicus* strain is disclosed in the European Patent No. 0 777 726 B1.

One of the problems associated with formulating an enzyme, such as a transglutaminase, in liquid form, is the lack of stability of the formulation. Further, one of the disadvantages associated with the present liquid enzyme formulations is that they contain at least one preservative.

### BRIEF DESCRIPTION OF THE INVENTION

An object of the present invention is thus to provide a liquid enzyme formulation comprising at least one milk protein crosslinking and/or modifying enzyme selected from transglutaminase, tyrosinase or protein glutaminase, which is stable and can be stored for a time period required from a commercial formulation in a room temperature or in temperatures of a refrigerator and/or a freezer.

Another object of the present invention is to provide a liquid formulation comprising a transglutaminase, which is stable and can be stored for a time period required from a commercial formulation in a room temperature or in temperatures of a refrigerator and/or a freezer.

A further object of the present invention is to provide a method for the preparation of a stable, liquid enzyme formulation comprising at least one milk protein crosslinking and/or modifying enzyme selected from transglutaminase, tyrosinase or protein glutaminase.

An even further object of the present invention is to provide a method for the preparation of a stable, liquid formulation comprising a transglutaminase.

The objects of the invention are achieved by the formulations and methods set forth in the independent claims. Preferred embodiments of the invention are described in the dependent claims.

Other objects, details and advantages of the present invention will become apparent from the following detailed description and examples.

### DETAILED DESCRIPTION OF THE INVENTION

Milk protein crosslinking and/or modifying enzymes such as transglutaminase, laccase, tyrosinase, peroxidase, sulfhydryl oxidase and protein glutaminase catalyze milk protein modifications. There seems to be synergism within the action of these enzymes and further with the action of glucose oxidase. Without being bound by any theory glucose oxidase and/or peroxidase seem to catalyze reactions wherein oxygen is released through hydrogen peroxide formation. The oxygen can then catalyze (oxidate) crosslinking of tyrosinase.

Milk protein crosslinking and/or modifying enzymes such as transglutaminase, laccase, tyrosinase, peroxidase, sulfhydryl oxidase and protein glutaminase optionally together with glucose oxidase are used in the manufacture of processed fish, meat and egg products, pastes and pates, fruits, berries and vegetables, soy products, cereal products, bread and bakery products.

Following milk protein crosslinking and/or modifying enzymes are all relevant in food prosessing in dairy or other food categories.

Transglutaminases are a family of enzymes (EC 2.3.2.13) that catalyze the generation of covalent linkages between the glutamine and lysine amino acid residues present in the protein molecules. When linkages are formed, ammonia is released.

Laccases (EC 1.10.3.2) derived from fungi and bacteria, such as, fungus *Trametes hirsute,* catalyze the crosslinking between carbohydrates and proteins (oxidation of aromatic compounds and cysteine) with applications in food processing for reduction of allergenicity, for example.

Tyrosinases (EC 1.14.18.1) are enzymes which catalyzes the oxidation of phenols such as tyrosine, with applications in food processing for reduction of allergenicity, for example.

Peroxidases (EC 1.11.1.7) are a family of enzymes that catalyze the oxidation of aromatic compounds with applications in food processing for reduction of allergenicity, for example.

Sulfhydryl oxidase (EC 1.8.3.3) catalyzes the formation of disulfide bonds, oxidation of glutathione.

Protein glutaminase catalyzes the deamidation of protein bound glutamine, and glutamine is converted to glutamic acid.

Glucose oxidase catalyzes the formation of protein crosslinks and oxidative gelation of pentosans.

Milk protein crosslinking and/or modifying enzymes such as transglutaminase, laccase, tyrosinase, peroxidase, sulfhydryl oxidase and protein glutaminase are used in dairy industry to stabilize the structure of milk-based products. In addition to dairy industry, these enzymes are used in the manufacture of processed fish, meat and egg products, pastes and pates, fruits, berries and vegetables, soy products, cereal products, bread and bakery products. Accordingly, a liquid enzyme formulation is suited for the manufacture of dairy, processed fish, meat, egg, pastes and pates, fruits, berries and vegetables, soy, cereal, bread and bakery products, for example. The use of a liquid enzyme preparation would be more practical and advantageous than the use of powder formulation, especially in the industrial scale manufacturing.

Transglutaminases are most active in the pH range from 5.2 to 8. When a liquidized transglutaminase is stored at pH 5.2 or at pH 8, the enzyme loses its activity quickly. After storing a transglutaminase at pH 5.2 for 7 days at room temperature or in the temperature of a refrigerator, only half of the activity is left.

The invention is based on the finding that when a transglutaminase, tyrosinase or protein glutaminase is stored in a suspension of a polyol selected from glycerol and sorbitol, and water in the pH range from 4.4 to 5.1, its activity is remained moderately during storage at room temperature and excellently during storage in the temperatures of a refrigerator and/or a freezer. In addition, the invention is based on the finding that when a transglutaminase together with a protein glutaminase is stored in a suspension of glycerol and water at pH 4.6, the activity of the enzymes remained moderately during storage at room temperature and excellently during storage in the temperatures of a refrigerator and/or a freezer. Further, the invention is based on the finding that when a transglutaminase together with a protein glutaminase and tyrosinase is stored in a suspension of glycerol and water at pH 4.6, the activity of the enzymes remained moderately during storage at room temperature and excellently during storage in the temperatures of a refrigerator and/or a freezer. In addition, the liquid preparations of the present invention are also microbiologically stable during the storage at room temperature and in the temperatures of a refrigerator and/or a freezer. Further, it was unexpectedly found that liquid enzyme formulation of the present invention maintained its enzyme activity and microbiological purity (no microbial growth) without any preservatives in the formulation in the temperatures of a refrigerator and/or a freezer.

In one embodiment, the liquid enzyme formulation in polyol-water suspension has pH value within the range from 4.4 to 5.1. In another embodiment of the present invention, the pH of the enzyme formulation is within the range from 4.4 to 4.8. In one embodiment of the present invention, the pH of the enzyme formulation is 4.4. In another embodiment of the present invention, the pH of the enzyme formulation is 4.6. In a further embodiment of the present invention, the pH of the enzyme formulation is 4.8. In an even further embodiment of the present invention, the pH of the enzyme formulation is 5.1.

Polyols selected from glycerol and sorbitol are used in the liquid enzyme formulation of the present invention. Mixtures of the polyols are also operable.

The suspension of a polyol and water or a mixture of two polyols and water suitable for formulating the enzyme preparation of the present invention may contain glycerol and/or sorbitol from 25% up to 100%, preferably from 50% up to 100% (w/w%). In one embodiment of the present invention, the enzyme is dissolved into 25% polyol/75% water (w/w) suspension. In another embodiment of the present invention, the enzyme is dissolved into 50% polyol/ 50% water (w/w) suspension. In a further embodiment of the present invention, the enzyme is dissolved into 75% polyol/25% water (w/w) suspension.

The suspension of glycerol and water suitable for formulating the enzyme preparation of the present invention may contain glycerol from 25% up to 100%, preferably from 50% up to 100%. In one embodiment of the present invention, the enzyme is dissolved into 25% glycerol/75% water suspension. In another embodiment of the present invention, the enzyme is dissolved into 50% glycerol/ 50% water suspension. In a further embodiment of the present invention, the enzyme is dissolved into 75% glycerol/25% water suspension. Further, the suspension of sorbitol and water suitable for formulating the enzyme preparation of the present invention may contain sorbitol from 25% up to 100%, preferably from 50% up to 100%. In one embodiment of the present invention, the enzyme is dissolved into 25% sorbitol/75% water suspension. In another embodiment of the present invention, the enzyme is dissolved into 50% sorbitol/50% water suspension. In a further embodiment of the present invention, the enzyme is dissolved into 75% sorbitol/25% water suspension.

The pH of the suspension may be adjusted to the desired range with an acid approved for food use, such as, lactic acid, GDL (glucono-deltalactone), citric acid, acetic acid, oxalic acid, malic acid, pantothenic acid, propionic acid and/or hydrochloric acid, or any mixtures/combinations thereof in the form of acid or salt. In one embodiment of the present invention, lactic acid is used for the pH adjustment.

The liquid enzyme preparation of the present invention may optionally contain also a preservative such as Na-benzoate. In one embodiment, the liquid enzyme preparation of the present invention does not contain any additional preservatives i.e., the formulation is free from preservatives. In another embodiment, the liquid enzyme preparation of the present invention contains an additional preservative. In a further embodiment, the liquid enzyme preparation of the present invention contains Na-benzoate as a preservative. In an even further embodiment, the liquid enzyme preparation of the present invention contains Na-benzoate, in an amount of 0.1 to 1%, preferably in an amount of 0.7% as a preservative.

The liquid enzyme preparation of the present invention maintains its activity in room temperature for about two weeks, in a refrigerator for about 1.5 to six months, at least and in freezer from a minimum of 5 months up to 24 months.

In one embodiment of the invention, the liquid formulation comprises one milk protein crosslinking and/or modifying enzyme. In another embodiment of the invention, the liquid formulation comprises two or more milk protein crosslinking and/or modifying enzymes. In one embodiment, the milk protein crosslinking and/or modifying enzyme in the formulation of the present invention is transglutaminase. In another embodiment, the milk protein crosslinking and/or modifying enzyme in the formulation of the present invention is tyrosinase. In another embodiment, the milk protein crosslinking and/or modifying enzyme in the formulation of the present invention is protein glutaminase. In another embodiment, the milk protein crosslinking and/or modifying enzymes in the formulation of the present invention are transglutaminase and protein glutaminase. In another embodiment, the milk protein crosslinking and/or modifying enzymes in the formulation of the present invention are transglutaminase and tyrosinase. In another embodiment, the milk protein crosslinking and/or modifying enzymes in the formulation of the present invention are transglutaminase and laccase. In a further embodiment, the milk protein crosslinking and/or modifying enzymes in the formulation of the present invention are transglutaminase, protein glutaminase and laccase. In an even further embodiment, the milk protein crosslinking and/or modifying enzymes in the formulation of the present invention are transglutaminase, protein glutaminase and tyrosinase.

The present invention relates also to a method for preparing a liquid enzyme formulation wherein at least one milk protein crosslinking and/or modifying enzyme selected from transglutaminase, tyrosinase or protein glutaminase is added to polyol-water suspension comprising from 25% to 100%(w/w) polyol selected from glycerol and sorbitol and having pH value within the range from 4.4 to 5.1. In one embodiment of the present invention, the pH of the polyol-water suspension is adjusted to a value within the range from 4.4 to 4.8. In one embodiment of the present invention, the pH of the enzyme formulation is adjusted to pH 4.4. In another embodiment of the present invention, the pH of the polyol-water suspension is adjusted to pH 4.6. In a further embodiment of the present invention, the pH of the polyol-water suspension is adjusted to pH 4.8. In an even further embodiment of the present invention, the pH of the enzyme formulation is adjusted to pH 5.1.

In one embodiment of the present invention, the enzyme is dissolved into 25% polyol-water suspension. In another embodiment of the present invention, the enzyme is dissolved into 50% polyol-water suspension. In a further embodiment of the present invention, the enzyme is dissolved into 75% polyol-water suspension. In one embodiment of the invention, the polyol is glycerol. In another embodiment of the invention, the polyol is sorbitol.

In the present method, the pH of the suspension may be adjusted to the desired range with an acid approved for food use (food grade, GRAS), such as, lactic acid, GDL, citric acid, acetic acid, oxalic acid, malic acid, pantothenic acid, propionic acid and/or hydrochloric acid or any mixtures/combinations thereof in the form of acid or salt. In one embodiment, lactic acid is used in the method of the present invention for the pH adjustment.

In the method of the present invention, also a preservative, such as Na-benzoate, may optionally be included in the liquid enzyme formulation. In one embodiment, the method of the present invention does not comprise addition of a preservative. In another embodiment, the method of the present invention comprises an addition of a preservative. In a further embodiment, the method of the present invention comprises addition of Na-benzoate as a preservative. In an even further embodiment, the method of the present invention comprises addition of Na-benzoate, in an amount of 0.1 to 1%, preferably in an amount of 0.7% as a preservative.

In one embodiment, the method of the present invention comprises the following steps:
a) pH of a polyol-water suspension is adjusted with food grade acid(s) to a value within the range from 4.4 to 5.1
b) a milk protein crosslinking and/or modifying enzyme is added to the suspension
c) optionally a preservative is added.

In another embodiment, the method of the present invention comprises the following steps:
a) a milk protein crosslinking and/or modifying enzyme is added to a polyol-water suspension
b) pH of the suspension is adjusted with food grade acid(s) to a value within the range from 4.4 to 5.1
c) optionally a preservative is added.

In one embodiment of the present invention, one milk protein crosslinking and/or modifying enzyme is added to the suspension. In another embodiment of the present invention, two or more milk protein crosslinking and/or modifying enzymes are added to the suspension. In one embodiment, the milk protein crosslinking and/or modifying enzyme is transglutaminase. In another embodiment, the milk protein crosslinking and/or modifying enzyme is tyrosinase. In another embodiment, the milk protein crosslinking and/or modifying enzyme is protein glutaminase. In another embodiment, the milk protein crosslinking and/or modifying enzymes are transglutaminase and protein glutaminase. In another embodiment, the milk protein crosslinking and/or modifying enzymes are transglutaminase and tyrosinase. In another embodiment, the milk protein crosslinking and/or modifying enzymes are transglutaminase and laccase. In a further embodiment, the milk protein crosslinking and/or modifying enzymes are transglutaminase, protein glutaminase and laccase. In an even further embodiment, the milk protein crosslinking and/or modifying enzymes are transglutaminase, protein glutaminase and tyrosinase.

The invention will be described in more detail by means of the following examples. The examples are not to be construed to limit the claims in any manner whatsoever.

### EXAMPLES

### COMPARATIVE EXAMPLE 1

### Transglutaminase preparation Activa® TG (Ajinomoto) in glycerol-water suspension at pH 5.2 at 4°C

The transglutaminase derived from Streptoverticillium mobaraense-strain having activity of 16.300 nkat/g (Activa® TG, Ajinomoto), was dissolved in activity of 274 nkat/g into 50% glycerol-water (w/w) suspension which pH was adjusted to 5.2 with lactic acid. The enzyme activity was monitored for 7 days. Additionally, the microbiological purity of the preparation was monitored.

On day 7, only 50% of the activity of the transglutaminase was left. No microbial growth was detected during the seven day preservation test.

### COMPARATIVE EXAMPLE 2

### Transglutaminase preparation Activa® TG (Ajinomoto) in water at pH 5.2 at 4°C

The transglutaminase Activa® TG (Ajinomoto) was dissolved in activity of 274 nkat/g into water having pH 5.2 adjusted with lactic acid. The suspension contained also 0.7% Na-benzoate as a preservative.

The enzyme activity was monitored for 50 days. Additionally, the microbiological purity of the preparation was monitored.

On day 50, only 43% of the activity of the transglutaminase was left. No microbial growth was detected during the 50 day preservation test.

### EXAMPLE 1

### Transglutaminase preparation Activa® TG (Ajinomoto) in glycerol-water suspension at pH 4.6 at 4°C

The transglutaminase Activa® TG (Ajinomoto) was dissolved in activity of 274 nkat/g into 50% glycerol-water (w/w) suspension having pH 4.6 adjusted with lactic acid.

The enzyme activity was monitored for 7 days. Additionally, the microbiological purity of the preparation was monitored.

On day 7, 100% of the activity of the transglutaminase was left. No microbial growth was detected during the seven day preservation test.

### EXAMPLE 2

### Transglutaminase preparation Activa® TG (Ajinomoto) in glycerol-water suspension at pH 4.6 at 4°C

The transglutaminase Activa® TG (Ajinomoto) was dissolved in activity of 326 nkat/g into 50% glycerol-water (w/w) suspension having pH 4.6 adjusted with lactic acid. The suspension contained also 0.7% Na-benzoate as a preservative.

The enzyme activity was monitored for 50 days. Additionally, the microbiological purity of the preparation was monitored.

On day 50, 100% of the activity of the transglutaminase was left. No microbial growth was detected during the 50 day preservation test.

### EXAMPLE 3

### Transglutaminase preparation Activa® TG-YG (Ajinomoto) in glycerol-water suspension at pH 4.6 at 4°C

The liquid transglutaminase formulation was prepared by dissolving Ajinomoto's transglutaminase preparation Activa® TG-YG, which contains a transglutaminase derived from Streptoverticillium mobaraense-strain and glutathione into 50% glycerol-water (w/w) suspension having pH 4.6 adjusted with lactic acid.

The enzyme activity was monitored for 50 days. Additionally, the microbiological purity of the preparation was monitored.

On day 50, 100% of the activity of the transglutaminase was left. No microbial growth was detected during the 50 day preservation test.

### EXAMPLE 4

### Transglutaminase preparation Activa® TG (Ajinomoto) in glycerol-water suspension at pH 4.4 at 4°C

The liquid transglutaminase preparation was prepared by dissolving transglutaminase Activa® TG (Ajinomoto) in activity of 274 nkat/g into 50% glycerol-water (w/w) suspension which pH was adjusted with lactic acid to pH 4.4.

The enzyme activity was monitored for 50 days. Additionally, the microbiological purity of the preparation was monitored.

On day 50, 100% of the activity of the transglutaminase was left. No microbial growth was detected during the 50 day preservation test.

### EXAMPLE 5

### Transglutaminase preparation Activa® TG (Ajinomoto) in glycerol-water suspension at pH 4.8 at 4°C

The liquid transglutaminase preparation was prepared by dissolving transglutaminase Activa® TG (Ajinomoto) in activity of 274 nkat/g into 50% glycerol-water (w/w) suspension which pH was adjusted with lactic acid to pH 4.8.

The enzyme activity was monitored for 50 days. Additionally, the microbiological purity of the preparation was monitored.

On day 50, 100% of the activity of the transglutaminase was left. No microbial growth was detected during the 50 day preservation test.

### EXAMPLE 6

### Transglutaminase preparation Activa® TG (Ajinomoto) in glycerol-water suspension at pH 5.1 at 4°C

The liquid transglutaminase preparation was prepared by dissolving transglutaminase Activa® TG (Ajinomoto) in activity of 274 nkat/g into 50% glycerol-water (w/w) suspension which pH was adjusted with lactic acid to pH 5.1 at 4°C.

The enzyme activity was monitored for 50 days. Additionally, the microbiological purity of the preparation was monitored.

On day 50, 100% of the activity of the transglutaminase was left. No microbial growth was detected during the 50 day preservation test.

### EXAMPLE 7

### Transglutaminase preparation Saprona TG (Yiming Biological Products Co, China) in glycerol-water suspension at pH 4.6 at 4°C

The liquid transglutaminase preparation was prepared by dissolving transglutaminase Yiming Saprona TG derived from Streptoverticillium mobaraense-strain into 50% glycerol-water (w/w) suspension which pH was adjusted with lactic acid to pH 4.6.

The enzyme activity was monitored for 50 days. Additionally, the microbiological purity of the preparation was monitored.

On day 50, 100% of the activity of the transglutaminase was left. No microbial growth was detected during the 50 day preservation test.

### EXAMPLE 8

### Transglutaminase preparation Reactyn CL 1000 TG (Campus SpA, Italy) in glycerol-water suspension at pH 4.6 at 4°C

The liquid transglutaminase preparation was prepared by dissolving transglutaminase Campus Reactyn CL 1000 TG into 50% glycerol-water (w/w) suspension which pH was adjusted with lactic acid to pH 4.6.

The enzyme activity was monitored for 50 days. Additionally, the microbiological purity of the preparation was monitored.

On day 50, 100% of the activity of the transglutaminase was left. No microbial growth was detected during the 50 day preservation test.

### EXAMPLE 9

### Transglutaminase preparation TG-PG (Ajinomoto) in glycerol-water suspension at pH 4.6 at 4°C

The liquid preparation was prepared by dissolving into 50% glycerol-water (w/w) suspension having pH 4.6 adjusted with lactic acid, an enzyme preparation TG-PG (Ajinomoto). The preparation TG-PG (Ajinomoto) contains a transglutaminase derived from *Streptoverticillium mobaraense-*strain and a protein glutaminase derived from *Chryseobacterium proteolyticum*.

The transglutaminase activity of the liquid preparation was 100 U/g and the protein glutaminase activity of the liquid preparation was 100 U/g.

The enzyme activity was monitored for 50 days. Additionally, the microbiological purity of the preparation was monitored.

On day 50, 100% of the activity of the transglutaminase and 100% of the activity of protein glutaminase were left. No microbial growth was detected during the 50 day preservation test.

### EXAMPLE 10

### Transglutaminase preparation Activa® TG (Ajinomoto) in 75% glycerol/25% water suspension at pH 4.6 at 4°C

The transglutaminase Activa® TG (Ajinomoto) was dissolved in activity of 326 nkat/g into 75% glycerol/25%water (w/w) suspension having pH 4.6 adjusted with lactic acid.

The enzyme activity was monitored for 50 days at the temperature of 4°C. Additionally, the microbiological purity of the preparation was monitored.

On day 50, 100% of the activity of the transglutaminase was left. No microbial growth was detected during the preservation test.

### EXAMPLE 11

### Transglutaminase preparation Activa® TG (Ajinomoto) in 25% glycerol/75% water suspension at pH 4.6 at 4°C

The transglutaminase Activa® TG (Ajinomoto) was dissolved in activity of 326 nkat/g into 25% glycerol/75%water (w/w) suspension having pH 4.6 adjusted with lactic acid.

The enzyme activity was monitored for 50 days at the temperature of 4°C. Additionally, the microbiological purity of the preparation was monitored.

On day 50, 72% of the activity of the transglutaminase was left. No microbial growth was detected during the preservation test.

### EXAMPLE 12

### Transglutaminase preparation Activa® TG (Ajinomoto) in glycerol-water suspensions at pH 4.4 - 4.8 at 22°C

The liquid transglutaminase preparations were prepared by dissolving transglutaminase Activa® TG (Ajinomoto) in activity of 2789 nkat/g into 50% glycerol-water (w/w) suspension which pH was adjusted with lactic acid to pH 4.4 - 4.8.

The enzyme activity of the preparations was monitored for 13 weeks at the temperature of 22°C. Additionally, the microbiological purity of the preparations was monitored.

After two weeks storage, the activity of the enzyme in the preparations started decreasing. No microbial growth was detected during the preservation test.

### EXAMPLE 13

### Transglutaminase preparation Activa® TG (Ajinomoto) in glycerol-water suspensions at pH 4.4 - 4.8 at 4°C

The liquid transglutaminase preparations were prepared by dissolving transglutaminase Activa® TG (Ajinomoto) in activity of 2789 nkat/g into 50% glycerol-water (w/w) suspension which pH was adjusted with lactic acid to pH 4.4 - 4.8.

The enzyme activity of the preparations was monitored for 26 weeks at the temperature of 4°C. Additionally, the microbiological purity of the preparations was monitored.

After 26 weeks storage, 89% of the activity of the enzyme was left. No microbial growth was detected during the preservation test.

### EXAMPLE 14

### Transglutaminase preparation Activa® TG (Ajinomoto) in glycerol-water suspensions at pH 4.4 - 4.8 at -20°C

The liquid transglutaminase preparations were prepared by dissolving transglutaminase Activa® TG (Ajinomoto) in activity of 2789 nkat/g into 50% glycerol-water (w/w) suspension which pH was adjusted with lactic acid to pH 4.4 - 4.8.

The enzyme activity of the preparations was monitored for 26 weeks at the temperature of -20°C. Additionally, the microbiological purity of the preparations was monitored.

After 26 weeks storage, 97% of the activity of the enzyme was left. No microbial growth was detected during the preservation test.

### EXAMPLE 15

### Tyrosinase preparation in glycerol-water suspension at pH 4.6 at 4°C

The tyrosinase enzyme was dissolved in activity of 100 U/g into 50% glycerol-water (w/w) suspension which pH was adjusted with lactic acid to pH 4.6.

The enzyme activity was monitored for 50 days. Additionally, the microbiological purity of the preparation was monitored.

After 50 days storage, 97% of the activity of the tyrosinase was left. No microbial growth was detected during the preservation test.

### EXAMPLE 16

### Protein glutaminase preparation in glycerol-water suspension at pH 4.6 at 4°C

The protein glutaminase was dissolved in activity of 100 U/g into 50% glycerol-water (w/w) suspension which pH was adjusted with lactic acid to pH 4.6.

The enzyme activity was monitored for 50 days. Additionally, the microbiological purity of the preparation was monitored.

After 50 days storage, 96% of the activity of the protein glutaminase was left. No microbial growth was detected during the preservation test.

### EXAMPLE 17

### Transglutaminase preparation Activa® TG (Ajinomoto) in sorbitol-water suspension at pH 4.6 at 4°C

The liquid transglutaminase preparation was prepared by dissolving transglutaminase Activa® TG (Ajinomoto) in activity of 274 nkat/g into 50% sorbitol-water (w/w) suspension which pH was adjusted with lactic acid to pH 4.6.

The enzyme activity was monitored for 50 days. Additionally, the microbiological purity of the preparation was monitored.

On day 50, 100% of the activity of the transglutaminase was left. No microbial growth was detected during the 50 day preservation test.

### EXAMPLE 18

### Liquid enzyme preparation containing TG-PG (Ajinomoto) and tyrosinase in glycerol-water suspension at pH 4.6 at 4°C

The liquid preparation was prepared by dissolving into 50% glycerol-water (w/w) suspension having pH 4.6 adjusted with lactic acid, an enzyme preparation TG-PG (Ajinomoto) and tyrosinase. The transglutaminase activity of the liquid preparation was 100 U/g, the protein glutaminase activity of the liquid preparation was 100 U/g and tyrosinase activity of the liquid preparation was 100 U/g.

The enzyme activity was monitored for 50 days. Additionally, the microbiological purity of the preparation was monitored.

On day 50, 100% of the activity of the transglutaminase, 100% of the activity of protein glutaminase and 98% of the activity of tyrosinase were left. No microbial growth was detected during preservation test.

It will be obvious to a person skilled in the art that, as the technology advances, the inventive concept can be implemented in various ways. The invention and its embodiments are not limited to the examples described above but may vary within the scope of the claims.

## Claims

1. A liquid enzyme formulation, **characterized in that** it comprises at least one milk protein crosslinking and/or modifying enzyme selected from transglutaminase, tyrosinase or protein glutaminase in polyol-water suspension comprising from 25% to 100% (w/w) polyol selected from glycerol and sorbitol, and having pH value within the range from 4.4 to 5.1.

2. A method for preparing a liquid enzyme formulation, **characterized in that** at least one milk protein crosslinking and/or modifying enzyme selected from transglutaminase, tyrosinase or protein glutaminase is added to polyol-water suspension comprising from 25% to 100% (w/w) polyol selected from glycerol and sorbitol, and having pH value within the range from 4.4 to 5.1.

3. The formulation according to claim 1 or the method according to claim 2, **characterized in that** the polyol-water suspension comprises from 50% to 75% polyol.

4. The formulation or method according to any one of the preceding claims, **characterized in that** the pH is 4.6.

5. The formulation or method according to any one of the preceding claims, **characterized in that** the formulation comprises transglutaminase and protein glutaminase.

6. The formulation or the method according to any one of the preceding claims, **characterized in that** the formulation comprises also laccase and/or tyrosinase.

7. The formulation or method according to any one of the preceding claims, **characterized in that** the formulation is free from preservatives.

8. The method according to any one of claims 2 to 7, **characterized in that** the method comprises the following steps:
a) pH of the polyol-water suspension is adjusted with food grade acid to a value within the range from 4.4 to 5.1,
b) at least one milk protein crosslinking and/or modifying enzyme is added to the suspension,
c) optionally a preservative is added.

9. The method according to any one of claims 2 to 7, **characterized in that** the method comprises the following steps:
a) at least one milk protein crosslinking and/or modifying enzyme is added to a polyol-water suspension comprising from 25% to 100% polyol,
b) pH of the suspension is adjusted with food grade acid(s) to a value within the range from 4.4 to 5.1
c) optionally a preservative is added.

10. A method for maintaining the activity of at least one milk protein crosslinking and/or modifying enzyme selected from transglutaminase, tyrosinase or protein glutaminase, in a liquid enzyme formulation, **characterized in that** the method comprises the following steps:
a) adding polyol to an aqueous enzyme solution to provide a polyol-water suspension comprising from 25% to 100% polyol selected from glycerol and sorbitol, and
b) adjusting the pH of the polyol-water suspension to a value within the range from 4.4 to 5.1.

11. The method according to claim 10, **characterized in that** the pH is adjusted to a value of 4.6.

12. The method according to claim 10 or 11, **characterized in that** the formulation is free from preservatives.

## Patentansprüche

1. Flüssige Enzymformulierung, **dadurch gekennzeichnet, dass** sie wenigstens ein Milchprotein vernetzendes und/oder modifizierendes Enzym, ausgewählt aus Transglutaminase, Tyrosinase oder Proteinglutaminase, in Polyol-Wasser-Suspension umfasst, die 25 % bis 100 % (Gew.-/Gew.) Polyol, ausgewählt aus Glycerol und Sorbitol, umfasst, und einen pH-Wert im Bereich von 4,4 bis 5,1 aufweist.

2. Verfahren zum Herstellen einer flüssigen Enzymformulierung, **dadurch gekennzeichnet, dass** wenigstens ein Milchprotein vernetzendes und/oder modifizierendes Enzym, ausgewählt aus Transglutaminase, Tyrosinase oder Proteinglutaminase, zu einer Polyol-Wasser-Suspension zugegeben wird, die 25 % bis 100 % (Gew.-/Gew.) Polyol, ausgewählt aus Glycerol und Sorbitol, umfasst, und einen pH-Wert im Bereich von 4,4 bis 5,1 aufweist.

3. Formulierung gemäß Anspruch 1 oder Verfahren gemäß Anspruch 2, **dadurch gekennzeichnet, dass** die Polyol-Wasser-Suspension 50 % bis 75 % Polyol umfasst.

4. Formulierung oder Verfahren gemäß einem der vorangehenden Ansprüche, **dadurch gekennzeichnet, dass** der pH 4,6 beträgt.

5. Formulierung oder Verfahren gemäß einem der vorangehenden Ansprüche, **dadurch gekennzeichnet, dass** die Formulierung Transglutaminase und Proteinglutaminase umfasst.

6. Formulierung oder Verfahren gemäß einem der vorangehenden Ansprüche, **dadurch gekennzeichnet, dass** die Formulierung auch Laccase und/oder Tyrosinase umfasst.

7. Formulierung oder Verfahren gemäß einem der vorangehenden Ansprüche, **dadurch gekennzeichnet, dass** die Formulierung frei von Konservierungsmitteln ist.

8. Verfahren gemäß einem der Ansprüche 2 bis 7, **dadurch gekennzeichnet, dass** das Verfahren die folgenden Schritte umfasst:
a) der pH der Polyol-Wasser-Suspension wird mit Säure in Lebensmittelqualität auf einen Wert im Bereich von 4,4 bis 5,1 eingestellt,
b) wenigstens ein Milchprotein vernetzendes und/oder modifizierendes Enzym wird zu der Suspension zugegeben,
c) gegebenenfalls wird ein Konservierungsmittel zugegeben.

9. Verfahren gemäß einem der Ansprüche 2 bis 7, **dadurch gekennzeichnet, dass** das Verfahren die folgenden Schritte umfasst:
a) wenigstens ein Milchprotein vernetzendes und/oder modifizierendes Enzym wird zu einer Polyol-Wasser-Suspension zugegeben, die 25 % bis 100 % Polyol umfasst,
b) der pH der Suspension wird mit Säure(n) in Lebensmittelqualität auf einen Wert im Bereich von 4,4 bis 5,1 eingestellt,
c) gegebenenfalls wird ein Konservierungsmittel zugegeben.

10. Verfahren zum Aufrechterhalten der Aktivität von wenigstens einem Milchprotein vernetzenden und/oder modifizierenden Enzym, ausgewählt aus Transglutaminase, Tyrosinase oder Proteinglutaminase, in einer flüssigen Enzymformulierung, **dadurch gekennzeichnet, dass** das Verfahren die folgenden Schritte umfasst:
a) Zugeben von Polyol zu einer wässrigen Enzymlösung, um eine Polyol-Wasser-Suspension bereitzustellen, die 25 % bis 100 % Polyol, ausgewählt aus Glycerol und Sorbitol, umfasst, und
b) Einstellen des pHs der Polyol-Wasser-Suspension auf einen Wert im Bereich von 4,4 bis 5,1.

11. Verfahren gemäß Anspruch 10, **dadurch gekennzeichnet, dass** der pH auf einen Wert von 4,6 eingestellt wird.

12. Verfahren gemäß Anspruch 10 oder 11, **dadurch gekennzeichnet, dass** die Formulierung frei von Konservierungsmitteln ist.

## Revendications

1. Formulation liquide d'enzyme, **caractérisée en ce qu'**elle comprend au moins une enzyme de réticulation et/ou de modification d'une protéine de lait sélectionnée parmi la transglutaminase, la tyrosinase ou la protéine glutaminase en suspension polyol-eau comprenant de 25 % à 100 % (en pds/pds) de polyol sélectionné parmi le glycérol et le sorbitol, et présentant une valeur de pH située dans la plage de 4,4 à 5,1.

2. Procédé de préparation d'une formulation liquide d'enzyme, **caractérisé en ce qu'**au moins une enzyme de réticulation et/ou de modification d'une protéine de lait sélectionnée parmi la transglutaminase, la tyrosinase ou la protéine glutaminase est ajoutée à la suspension polyol-eau comprenant de 25 % à 100 % (en pds/pds) de polyol sélectionné parmi le glycérol et le sorbitol, et présentant une valeur de pH située dans la plage de 4,4 à 5,1.

3. Formulation selon la revendication 1 ou procédé selon la revendication 2, caractérisé-e en ce que la suspension polyol-eau comprend de 50 % à 75 % de polyol.

4. Formulation ou procédé selon l'une quelconque des revendications précédentes, caractérisé-e en ce que le pH est de 4,6.

5. Formulation ou procédé selon l'une quelconque des revendications précédentes, caractérisé-e en ce que la formulation comprend de la transglutaminase et de la protéine glutaminase.

6. Formulation ou procédé selon l'une quelconque des revendications précédentes, caractérisé-e en ce que la formulation comprend également de la laccase et/ou tyrosinase.

7. Formulation ou procédé selon l'une quelconque des revendications précédentes, caractérisé-e en ce que la formulation est exempte d'agents conservateurs.

8. Procédé selon l'une quelconque des revendications 2 à 7, **caractérisé en ce que** le procédé comprend les étapes suivantes :
a) le pH de la suspension polyol-eau est ajusté avec de l'acide de qualité alimentaire à une valeur située dans la plage de 4,4 à 5,1,
b) au moins une enzyme de réticulation et/ou de modification d'une protéine de lait est ajoutée à la suspension,
c) éventuellement un agent conservateur est ajouté.

9. Procédé selon l'une quelconque des revendications 2 à 7, **caractérisé en ce que** le procédé comprend les étapes suivantes :
a) au moins une enzyme de réticulation et/ou de modification d'une protéine de lait est ajoutée à la suspension polyol-eau comprenant de 25 % à 100 % de polyol,
b) le pH de la suspension est ajusté avec un(des) acide(s) de qualité alimentaire à une valeur située dans la plage de 4,4 à 5,1
c) éventuellement un agent conservateur est ajouté.

10. Procédé de maintien de l'activité d'au moins une enzyme de réticulation et/ou de modification d'une protéine de lait sélectionnée parmi la transglutaminase, la tyrosinase ou la protéine glutaminase, dans une formulation liquide d'enzyme, **caractérisé en ce que** le procédé comprend les étapes suivantes :
a) d'addition de polyol à une solution aqueuse d'enzyme pour fournir une suspension polyol-eau comprenant de 25 % à 100 % de polyol sélectionné parmi le glycérol et le sorbitol, et
b) d'ajustement du pH de la suspension polyol-eau à une valeur située dans la plage de 4,4 à 5,1.

11. Procédé selon la revendication 10, **caractérisé en ce que** le pH est ajusté à une valeur de 4,6.

12. Procédé selon la revendication 10 ou 11, **caractérisé en ce que** la formulation est exempte d'agents conservateurs.
